Europäisches Patentamt

European Patent Office

Office européen des brevets

(11) Publication number: 0 346 798
A1

## (12) EUROPEAN PATENT APPLICATION

(21) Application number: 89110593.4

(22) Date of filing: 12.06.89

(51) Int. Cl.⁴: A61K 37/36 , //(A61K37/36, 31:44,31:27,31:14)

(30) Priority: 16.06.88 IT 2098488

(43) Date of publication of application:
20.12.89 Bulletin 89/51

(84) Designated Contracting States:
AT BE CH DE ES FR GB GR IT LI LU NL SE

(71) Applicant: PIERREL S.p.A.
Via De Pretis, 88
I-80100 Napoli(IT)

(72) Inventor: Boghen, Franklin Muny
Via Vignate, 94
I-27025 Gambolo' Pavia(IT)
Inventor: Camanni, Franco
Corso Chieri, 181
I-10100 Torino(IT)
Inventor: Ghigo, Ezio
Via Assietta, 17
I-10128 Torino(IT)
Inventor: Muller, Eugenio
Via Mangiagalli, 5
I-20133 Milano(IT)

(74) Representative: Gervasi, Gemma et al
NOTARBARTOLO & GERVASI Srl Viale
Bianca Maria 33
I-20122 Milan(IT)

(54) Use of a cholinergic agonist and growth hormone releasing hormone for preparing pharmaceutical compositions useful in the differential diagnosis of somatotropic deficiency.

(57) The use of a cholinergic agonist and growth hormone releasing hormone for the preparation of pharmaceutical compositions useful in the differential diagnosis of somatotropic deficiency.

EP 0 346 798 A1

# USE OF A CHOLINERGIC AGONIST AND GROWTH HORMONE RELEASING HORMONE FOR PREPARING PHARMACEUTICAL COMPOSITIONS USEFUL IN THE DIFFERENTIAL DIAGNOSIS OF SOMATOTROPIC DEFICIENCY

## Field of the invention

This invention relates to the use of a cholinergic agonist and growth hormone releasing hormone for preparing pharmaceutical compositions useful in the differential diagnosis of somatotropic deficiency, the term "cholinergic agonist" meaning either a directly acting substance or an anticholinesterase agent.

More specifically, this invention relates to the use of said active principles in the preparation of a pharmaceutical composition suitable for the simultaneous intravenous or intramuscular administration of a cholinergic agonist and GHRH.

In the present text, the following abbreviations will be used for greater clarity and simplicity: pyridostigmine = PD; growth hormone (somatotropin) = GH; growth hormone releasing hormone = GHRH; insulin hypoglycemia = IH; clonidine = CLON; central nervous system = SNC; PD + GHRH = simultaneous intravenous or intramuscular administration of the two substances; normal children = BN; children of low familial stature = BSF; children with constitutional growth retardation = RCC; true somatotropic deficiency = GHD.

## Prior art

The diagnosis of somatotropic deficiency in low-stature children with a reduced linear growth rate and skeletal retardation has for long been based on the determination of less than normal GH secretion following two or more separately administered pharmacological stimuli (such as arginine, insulin, levodopa, clonidine).

These criteria were applied up to a short time ago, when it was demonstrated that many low-stature children with a reduced linear growth rate and a retarded skeletal age, although showing normal somatotropic response to the aforesaid pharmacological stimuli, have reduced basal GH secretion over 24 hours (Spiliotis B.E., August G.P., Hung W., Mendelson W., Bercu B., JAMA, 22; 497-504, 1984).

It was thus demonstrated that these children have deficient somatotropic secretion which is due not to hypophyseal insufficiency but rather to SNC disorder and more specifically to disorder of the hypothalamic structures responsible for the production of GHRH (Evain-Brion D., "Neuroendocrine Perspectives", E.E. Müller and R.M. McLeod, Eds., vol. 5, pp 101-109 Elsevier Amsterdam, 1986).

GHRH is a peptide which was isolated by Guillemin et al. from the pancreas of a pancreatic tumour patient showing signs of acromegaly. Various forms of GHRH have been isolated and purified composed of 44, 40, 37 and 29 amino acids all possessing equal activity in stimulating GH release (Guillemin R., Brasean P., Bohlen P., Esch S., Ling N., Wehrenberg W., Growth Hormone Releasing Factor from a Human Pancreatic Tumour that caused Acromegaly, Science vol, 218, pp. 585-587, 1982).

Somatotropic deficiency due to hypothalamic disorder and consequent deficient GHRH secretion has been called "primary neurosecretory disorder" (Spiliotis B.E., August G.P., Hung W., Mendelson W., Bercu B., JAMA 22; 497-504, 1984).

This new concept clearly indicates that all traditional pharmacological tests used up to the present time are unable to correctly ascertain the existance of somatotropic deficiency as they can give false negative responses in normal subjects, and even more so are unable to effect a differential diagnosis between somatotropic deficiency due to hypophyseal insufficiency and somatotropic deficiency due to hypothalamic disorder, as they are unable to distinguish between them.

There is therefore a requirement for new pharmaceutical compositions able to effect reliable and precise diagnosis on all patients under all somatotropic deficiency conditions, and thus determine the most effective therapy for the diagnosed deficiency, either at the hypophyseal or hypothalamic level, as in the first case a therapy involving GH administration is suitable whereas in the second case chronic GHRH administration would be a more rational therapy.

The face that cholinergic agonists are able to increase the secretion of both basal GH and GH induced by GHRH in normal adults (Massara F., Ghigo E., Demislis K., Tangolo D., Mazza E. Locatelli V., Müller E.E., Molinatti G.M., Camanni F., Neuroendocrinology 43: 670-675, 1986) was the reason for studying the interactions between the cholinergic system and somatotropic secretion in a group of children.

A group of children was divided by normal auxological criteria into three smaller groups, namely children of low familial stature, children with constitutional growth retardation, and children with somatotropic deficiency (Ghigo E., Mazza E., Imperiale E., Rizzi G., Benso L., Müller E., Camanni F., Massara F., Journal of Clinical Endocrinology and Metabolism, vol. 65, pp. 452-456, 1987).

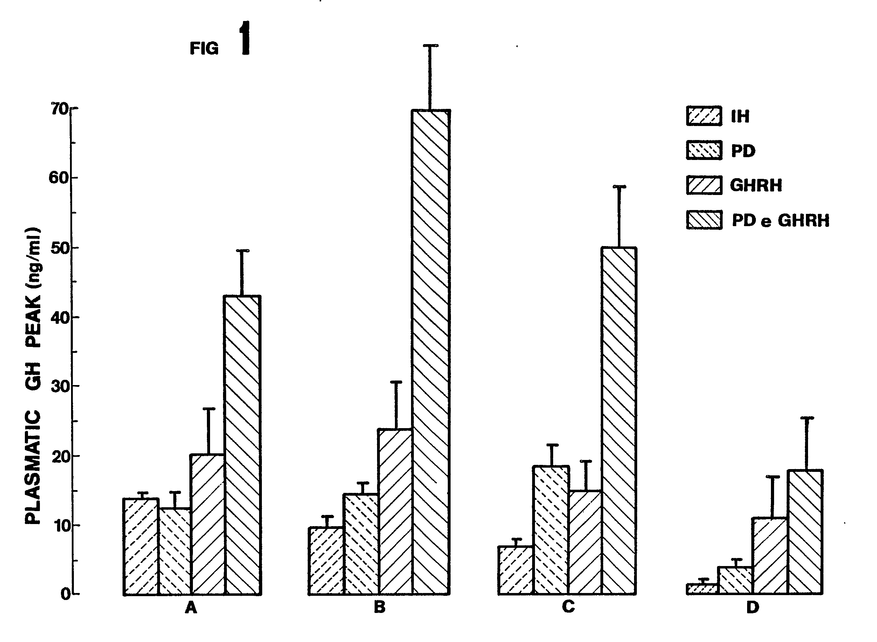

Figure 1 shows the results of this study compared with the secretory response induced by the same tests in normal children.

In the group of children suffering from GHD, somatotropic deficiency was confirmed by deficient GH release after hypoglycemia induced by insulin and/or other pharmacological tests, and by low somatomedin levels in the plasma (< 0.4 U/ml), which indicates GH deficiency. All the children were subjected to the following 4 tests: a) IH (0.15 IU/kg, iv); b) GHRH (1 μg/kg, iv bolus); c) PD (60 mg, oral); PD + GHRH (60 mg PD oral 60 minutes before, then 1 μg/kg iv of GHRH).

As shown in Figure 1, in all the 4 groups of children examined, BN (A), BSF (B), RCC (C), GHD (D), the increase in cholinergic tone induced by PD increased the GH levels in the plasma and potentiated the response to the subsequently administered GHRH.

When GHRH administration was preceded by PD administration, the GHRH always induced a somatotropic response higher than that induced by IH or GHRH alone.

## Summary of the invention

Pharmaceutical compositions for diagnostic use employed up to the present time for diagnosing somatotropic deficiency, including GHRH, have two substantial drawbacks: they frequently give false-negative responses in normal subjects and can thus lead to mistaken diagnosis of somatotropic deficiency in normal subjects, and in addition they are unable to differentiate between somatotropic deficiency due to hypophyseal insufficiency and somatotropic deficiency due to hypothalamic disorder, these deficiencies requiring different therapies.

We have now unexpectedly found that the use of a cholinergic agonist and GHRH in the preparation of pharmaceutical compositions according to the present invention completely obviates these drawbacks as it produces no false-negative responses in normal subjects and allows reliable differential diagnosis between the two types of somatotropic deficiency.

In order to demonstrate the efficiency of the pharmaceutical compositions of the present invention, the effect of cholinergic activation induced by PD on basal somatotropic secretion and on that stimulated by GHRH was compared with the results obtained by studying spontaneous nocturnal somatotropic secretion (from 23.00 to 07.00 hours) and somatotropic secretion induced by acute administration of GHRH and CLON, administered separately, on a large number of children, namely 8 normal children (Figure 2), 16 of low familial stature (Figure 3), 26 with constitutional growth retardation (Figure 4), 5 with somatotropic deficiency due to evident organic defect and 5 with idiopathic somatotropic deficiency (Figures 5a and 5b respectively).

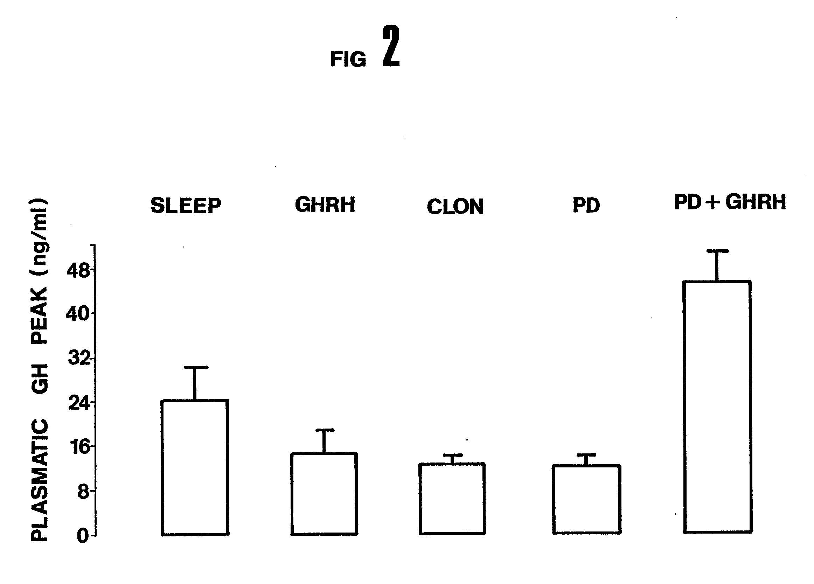

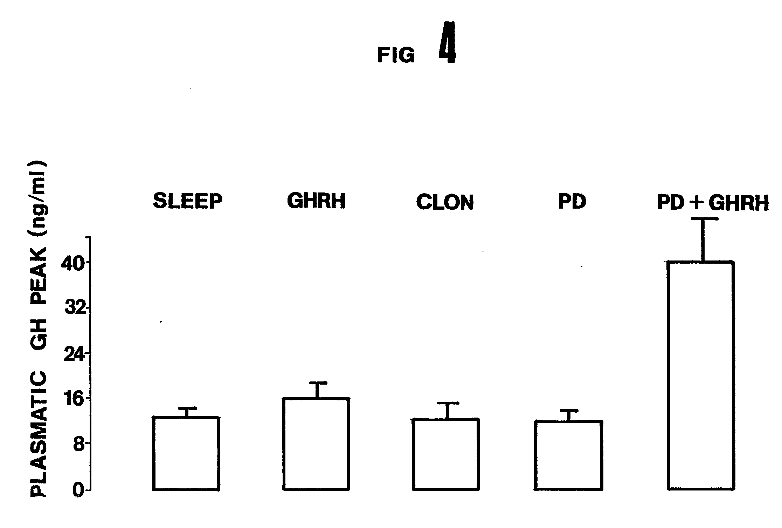

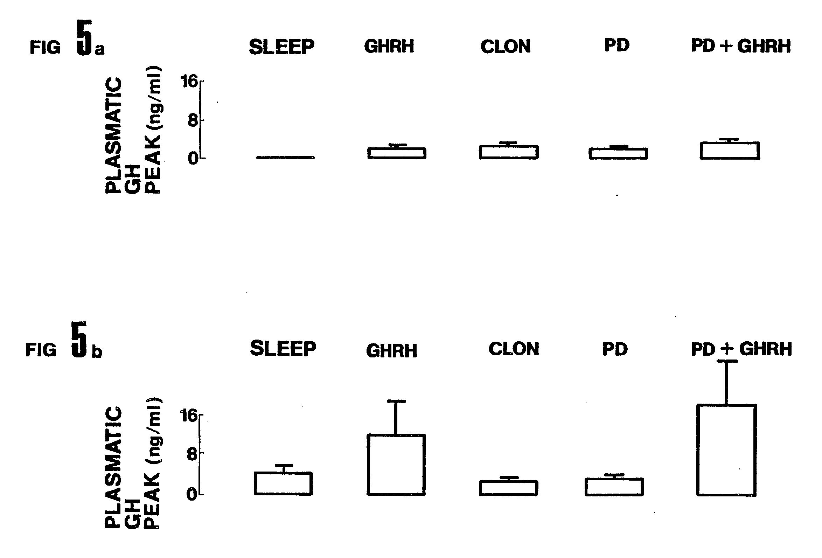

Figures 2 to 5 show the nocturnal plasmatic GH peak expressed in ng/ml, both under normal conditions during sleep and after treatment with GHRH, CLON, PD and PD + GHRH.

It is apparent that in all 5 treated groups of children, the spontaneous nocturnal secretion gives a curve which can be superimposed on that induced by GHRH, CLON and PD, and thus these tests cannot be used to make reliable diagnoses as they are difficult and uncertain to read.

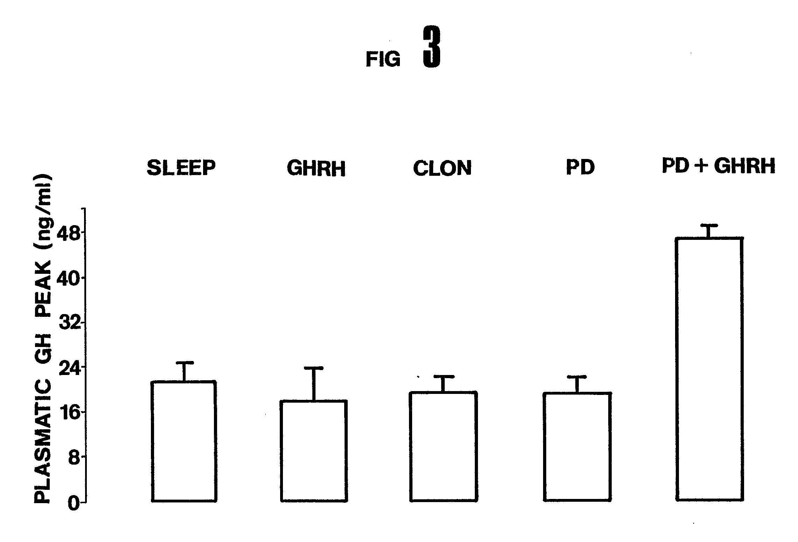

In contrast, GH secretion induced by PD + GHRH in 4 groups of treated children (Figures 3, 4, 5b) is considerably higher than the basal nocturnal secretion and therefore provides a precise indication useful to correct diagnosis of hypophyseal functionality.

Table 1 shows the results obtained in this study. The normal somatotropic response values assumed for the various tests were a plasmatic concentration peak of GH ≥ 10 ng/ml for the tests conducted by administering GHRH, CLON and PD, and a plasmatic concentration peak of GH ≥ 20 ng/ml for the test conducted by administering PD + GHRH.

To obtain the results given in this table the BN group, ie the group with normal GH secretion of average nocturnal GH concentration ≥ 3 ng/ml, was subjected to diagnostic evaluation with GHRH, CLON, PD and PD + GHRH, and it can be seen that a valid response is obtained only on administering PD + GHRH in that in all other tests there is a high percentage of false-negative responses (4/8, 2/8, 3/8) which would lead to a mistaken diagnosis of somatotropic deficiency also in normal subjects.

Only the PD + GHRH test confirmed in all normal subjects (8/8) the presence of somatotropic secretion normality, there being no false-negative response present, thus nullifying the risk of mistaken diagnosis in normal subjects.

Of all the 52 children examined pertaining to the BSF, RCC, idiopathic GHD and organic GHD groups, a normal average nocturnal concentration (≥ 3 ng/ml) was present only in 27 out of 52 subjects (13 BSF, 14 RCC).

In all these 27 children, administration of PD + GHRH induced normal secretion increase of GH ≥ 20 ng/ml, whereas with GHRH, CLON or PD administration the GH secretion increase frequently reached values lower than those necessary for the validity of the test, ie 10 ng/ml.

In children with average nocturnal GH secretion of below the norm (3 BSF, 12 RCC, all idiopathic and organic GHD), administration of PD + GHRH showed the existence of true hypophyseal insufficiency only in 4 RCC, in 3 idiopathic GHD and in all 5 organic GHD, so showing that the remaining children (16 BSF, 22 RCC and 2 GHD) had primary neurosecretory disorder and thus insufficiency only at hypothalamic level and not at hypophyseal level.

This test therefore gave reliable differential diagnosis of the two pathologies, which up to the present time has never been possible to do using traditional tests.

The results obtained show that combined administration of PD + GHRH represents the most powerful and reliable pharmacological test for ascertaining the somatotropic secretory capacity of hypophysis as there are no false-negative responses in normal children, as instead happens in all traditional tests used up to the present time.

Moreover, the use of a pharmaceutical composition containing PD + GHRH is able to distinguish GH deficiency due to a real impossibility of the hypophysis to secrete growth hormone (GH) from that due to functional alterations at SNC level, and in particular at hypothalamic level, and on the basis of this diagnosis enables the most suitable treatment for the identified pathology to be chosen with accuracy, ie whether to directly administer GH in cases of real hypophyseal insufficiency or to administer a pharmaceutical composition containing a cholinergic agonist and GHRH, or one of the normal therapies in use, in cases of functional alterations of the hypothalamus.

## Detailed description of the invention

The composition containing GHRH and a cholinergic agonist for use in the diagnosis of somatotropic deficiencies according to the present invention can be prepared using various types of cholinergic agonists and excipients. It can be further made up into various injectable formulations according to applicational requirements.

Usable cholinergic agonists include:
- choline esters such as carbachol;
- natural and synthetic alkaloids such as arecoline;
- reversible anticholinesterase agents such as neostigmine and pyridostigmine;
and their relative salts.

In addition to GHRH and the cholinergic agonist the composition according to the present invention can contain one or more pharmaceutically acceptable non-toxic excipients.

The choice of excipients depends not only on the chemical and physical characteristics of the active principles and the required posology, but also on the type of composition wanted.

The dosage of the individual components of the administration obviously varies as a function of the patient's body weight and clinical condition.

The typical dosage of GHRH is from 0.5 to 1.5 $\mu$g/kg whereas for the cholinergic agonist it is from 0.001 to 5 mg/kg.

More specifically, in pharmaceutical compositions according to the present invention for pediatric use, the pyridostigmine content can vary from 0.1 to 2.5 mg and preferably from 0.25 to 0.5 mg, the neostigmine content can vary from 0.05 to 2 mg and preferably from 0.10 to 0.5 mg, and the carbachol content can vary from 0.05 to 0.25 mg and preferably from 0.1 to 0.2 mg. In pharmaceutical compositions for adult use, the pyridostigmine content can vary from 0.20 to 5 mg and preferably from 0.5 to 1.5 mg, the neostigmine content can vary from 0.20 to 2 mg and preferably from 0.25 to 0.75 mg, and the carbachol content can vary from 0.1 to 2 mg and preferably from 0.2 to 0.5 mg.

The GHRH content of injectable solutions according to the present invention can vary from 0.001 to 0.75% w/w and the cholinergic agonist content can vary from 0.01 to 7.5% w/w.

The quantity of composition needed to release the desired dose obviously depends on the concentration of the active principles in the composition.

The compositions according to the present invention for intravenous or intramuscular injection can be prepared in the form of 2 or 3 vials depending on the cholinergic agonist present. If 2 vials are prepared the first contains lyophilised GHRH with suitable excipients, and the second contains the cholinergic agonist in aqueous solution with suitable excipients to dissolve the lyophilised product at the time of administration, whereas if 3 vials are prepared the first contains the GHRH as above described, the second contains the lyophilised cholinergic agonist with suitable excipients, and the third contains a suitable solvent for dissolving the two lyophilised products at the time of administration.

The following examples of the preparation of compositions according to the present invention are given by way of non-limiting illustration.

EXAMPLE 1

A. Pediatric use

An injectable solution consisting of a vial of lyophilised product and a vial of solvent for forming the solution at the time of intravenous or intramuscular injection:

| - lyophilised vial | | |
|---|---|---|
| GHRH | 30 | μg |
| glycine | 4.4 | mg |
| sodium phosphate | 0.08 | mg |
| mannitol | 50 | mg |

The mannitol, glycine and GHRH are dissolved in distilled water. The solution is divided equally into 100 vials, frozen and lyophilised.

At the time of use the solution for injection is prepared by adding to a vial 1 ml of a solution of the following composition:

| - solvent vial | | |
|---|---|---|
| pyridostigmine | 0.5 | mg |
| sodium acetate | 25 | mg |
| water for injectables to make up to | 1 | ml |

B. Adult use

As for pediatric use but with the following composition:

| - lyophilised vial | | |
|---|---|---|
| GHRH | 70 | μg |
| glycine | 4.4 | mg |
| sodium phosphate | 0.08 | mg |
| mannitol | 50 | mg |

| - solvent vial | |
|---|---|
| pyridostigmine | 1 mg |
| sodium acetate | 20 mg |
| water for injectables to make up to | 1 ml |

EXAMPLE 2

5

A. Pediatric use

A vial containing lyophilised product as described in Example 1 but containing 25 mg of mannitol is dissolved at the time of administration in the contents of a solvent vial having the following composition:

| - solvent vial | | |
|---|---|---|
| pyridostigmine | 0.5 | mg |
| sodium chloride | 4.2 | mg |
| sodium acetate | 20 | mg |
| water for injectables to make up to | 1 | ml |

B. Adult use

As for pediatric use but having the following composition:

| - lyophilised vial | | |
|---|---|---|
| GHRH | 70 | μg |
| glycine | 4.4 | mg |
| sodium phosphate | 0.08 | mg |
| mannitol | 25 | mg |

| - solvent vial | | |
|---|---|---|
| pyridostigmine | 1 | mg |
| sodium chloride | 4.2 | mg |
| sodium acetate | 15 | mg |
| water for injectables to make up to | 1 | ml |

EXAMPLES 3 and 4

A vial containing lyophilised product as described in Examples 1 and 2 is dissolved at the time of administration in the contents of a solvent vial having the following composition:

A. Pediatric use

| — solvent vial | | |
|---|---|---|
| carbachol | 0.1 | mg |
| sodium chloride | 9 | mg |
| water for injectables to make up to | 1 | ml |

B. Adult use

6

| - solvent vial | | |
|---|---|---|
| carbachol | 0.25 | mg |
| sodium chloride | 9 | mg |
| water for injectables to make up to | 1 | ml |

## EXAMPLE 5

A vial containing lyophilised product as described in Example 1 is dissolved at the time of administration in the contents of a solvent vial having the following composition:

A. Pediatric use

| - solvent vial | | |
|---|---|---|
| neostigmine | 0.1 | mg |
| sodium chloride | 3.5 | mg |
| water for injectables to make up to | 1 | ml |

B. Adult use

| - solvent vial | | |
|---|---|---|
| neostigmine | 0.5 | mg |
| sodium chloride | 2.5 | mg |
| water for injectables to make up to | 1 | ml |

## EXAMPLE 6

A vial containing lyophilised product as described in Example 2 is dissolved at the time of administration in the contents of a solvent vial having the following composition:

A. Pediatric use

| - solvent vial | | |
|---|---|---|
| neostigmine | 0.1 | mg |
| sodium chloride | 7 | mg |
| water for injectables to make up to | 1 | ml |

B. Adult use

| - solvent vial | | |
|---|---|---|
| neostigmine | 0.5 | mg |
| sodium chloride | 5 | mg |
| water for injectables to make up to | 1 | ml |

## EXAMPLE 7

A vial containing lyophilised product as described in Example 1 is dissolved at the time of administration in the contents of a solvent vial having the following composition:

### A. Pediatric use

| - solvent vial | | |
|---|---|---|
| arecholine | 0.1 | mg |
| sodium chloride | 3 | mg |
| water for injectables to make up to | 1 | ml |

### B. Adult use

| - solvent vial | | |
|---|---|---|
| arecholine | 0.3 | mg |
| sodium chloride | 1 | mg |
| water for injectables to make up to | 1 | ml |

## EXAMPLE 8

An injectable solution consisting of a vial of lyophilised product and a vial of solvent for forming the solution at the time of intravenous or intramuscular injection, with the characteristic that the composition of the vial of lyophilised product is as in Examples 1 to 7 but also containing in the lyophilised vial the cholinergic agonist of the respective example.

At the moment of use the solution for injection is formed by adding a vial containing 1 ml of water for injectable compositions.

TABLE 1

| GROUP | SLEEP ≥3 ng/ml | GHRH ≥10 ng/ml | CLON ≥10 ng/ml | PO ≥10 ng/ml | PD + GHRH ≥20 ng/ml | POSSIBLE THERAPEUTIC TREATMENT |
|---|---|---|---|---|---|---|
| Normal children | 8/8 | 4/8 | 6/8 | 5/8 | 8/8 | |
| Low familial stature | 13/16 | 5/8 | 12/16 | 14/16 | 16/16 | 16 = PD + GHRH |
| Constitutional growth retardation | 14/26 | 13/26 | 11/26 | 13/26 | 22/26 | 4 = GH 16 = PD + GHRH |
| Idiopathic somatotropic deficiency | 0/5 | 2/5 | 0/5 | 0/5 | 2/5 | 3 = GH 2 = PD + GHRH |
| Organic somatotropic deficiency | 0/5 27/52 (52%) | 0/5 | 0/5 | 0/5 | 0/5 | 5 = GH 40/52 (77%) |

## Claims

1. The use of a cholinergic agonist and growth hormone releasing hormone (GHRH) for the preparation of pharmaceutical compositions useful in the differential diagnosis of somatotropic deficiency.

2. The use of a cholinergic agonist and growth hormone releasing hormone (GHRH) as claimed in claim 1, characterised in that said cholinergic agonist is carbachol, arecholine, neostigmine, pyridostigmine or their salts.

3. The use of a cholinergic agonist and growth hormone releasing hormone (GHRH) as claimed in claim 1, characterised in that said composition can be prepared in injectable solution form.

4. The use of a cholinergic agonist and growth hormone releasing hormone (GHRH) as claimed in claim 3, characterised in that said solution can be injected intravenously or intramuscularly.

5. The use of a cholinergic agonist and growth hormone releasing hormone (GHRH) as claimed in claim 3, characterised in that the GHRH content of said solution is between 0.001 and 0.75% w/w and the cholinergic agonist content is between 0.01 and 7.5% w/w.

6. The use of a cholinergic agonist and growth hormone releasing hormone (GHRH) as claimed in claim 3, characterised in that said solution is prepared by mixing lyophilised GHRH with a suitable solvent containing the cholinergic agonist.

7. Diagnostic compositions for the differential diagnosis of somatotropic deficiency, characterised by comprising a cholinergic agonist and growth hormone releasing hormone (GHRH).

8. Diagnostic compositions for the differential diagnosis of somatotropic deficiency as claimed in claim 7, characterised in that said cholinergic agonist is carbachol, arecholine, neostigmine, pyridostigmine or their salts.

9. Diagnostic compositions for the differential diagnosis of somatotropic deficiency as claimed in claim 7, characterised in that said composition can be prepared in injectable solution form.

10. Diagnostic compositions for the differential diagnosis of somatotropic deficiency as claimed in claim 9, characterised in that said solution can be injected intravenously or intramuscularly.

11. Diagnostic compositions for the differential diagnosis of somatotropic deficiency as claimed in claim 9, characterised in that the GHRH content of said solution is between 0.001 and 0.75% w/w and the cholinergic agonist content is between 0.01 and 7.5% w/w.

12. Diagnostic compositions for the differential diagnosis of somatotropic deficiency as claimed in claim 9, characterised in that said solution is prepared by mixing lyophilised GHRH with a suitable solvent containing the cholinergic agonist.

FIG 1

PLASMATIC GH PEAK (ng/ml)

IH
PD
GHRH
PD e GHRH

A    B    C    D

EP 0 346 798 A1

FIG 2

EP 0 346 798 A1

EP 0 346 798 A1

FIG **3**

Bar chart showing PLASMATIC GH PEAK (ng/ml) for conditions SLEEP, GHRH, CLON, PD, and PD + GHRH.

FIG 4

PLASMATIC GH PEAK (ng/ml)

SLEEP  GHRH  CLON  PD  PD+GHRH

EP 0 346 798 A1

EP 0 346 798 A1

FIG **5**a

|  | SLEEP | GHRH | CLON | PD | PD + GHRH |

FIG **5**b

|  | SLEEP | GHRH | CLON | PD | PD + GHRH |

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,D | CHEMICAL ABSTRACTS, vol. 105, no. 13, 29th September 1986, page 502, abstract no. 113044x, Columbus, Ohio, US; F. MASSARA et al.: "Cholinergic involvement in the growth hormone releasing hormone-induced growth hormone release: studies in normal and acromegalic subjects", & NEUROENDOCRINOLOGY 1986, 43(6), 670-5 * Abstract * | 1-12 | A 61 K 37/36 // (A 61 K 37/36 A 61 K 31:44 A 61 K 31:27 A 61 K 31:14 ) |
| X | CHEMICAL ABSTRACTS, vol. 105, no. 21, 24th November 1986, page 113, abstract no. 184381r, Columbus, Ohio, US; F. MASSARA et al.: "Potentiation of cholinergic tone by pyridostigmine bromide re-instates and potentiates the growth hormone responsiveness to intermittent administration of growth hormone-releasing factor in man", & ACTA ENDOCRINOL. (COPENHAGEN) 1986, 113(1), 12-16 * Abstract * | 1-12 | |
| X | CHEMICAL ABSTRACTS, vol. 90, no. 3, 15th Jaunary 1979, page 45, abstract no. 16291q, Columbus, Ohio, US; J.F. BRUNI et al.: "Effects of cholinergic drugs on growth hormone release", & LIFE SCI. 1978, 23(13), 1351-7 * Abstract * | 1-12 | TECHNICAL FIELDS SEARCHED (Int. Cl.4) A 61 K |

The present search report has been drawn up for all claims

| Place of search | Date of completion of the search | Examiner |
|---|---|---|
| THE HAGUE | 25-09-1989 | BRINKMANN C. |

EPO FORM 1503 03.82 (P0401)